Europäisches Patentamt

**European Patent Office**

**Office européen des brevets**

(11) Veröffentlichungsnummer: **0 006 435**
**A1**

(12) **EUROPÄISCHE PATENTANMELDUNG**

(21) Anmeldenummer: **79101431.9**

(22) Anmeldetag: **10.05.79**

(51) Int. Cl.³: **A 61 K 31/495**
**// (A61K31/495, 31/48)**

(30) Priorität: **12.05.78 DE 2820937**

(43) Veröffentlichungstag der Anmeldung: **09.01.80**
**Patentblatt 80/1**

(84) Benannte Vertragsstaaten: **AT BE CH FR GB IT LU NL
SE**

(71) Anmelder: **DOLORGIET ARZNEIMITTELFABRIK
PETER DOLL KG, Koblenzer Strasse 112, D-5300
Bonn 2-Bad Godesberg (DE)**

(72) Erfinder: **Wagener, Hans Henrich, Prof. Dr. med.,
Breslauer Strasse 76, D-5309 Meckenheim (DE)**
Erfinder: **Emschermann, Bernhard, Dr.,
Sandtstrasse 55, D-5300 Bonn-Kessenich (DE)**
Erfinder: **Löhner, Manfred, Blücherstrasse 42, D-5300
Bonn 1 (DE)**
Erfinder: **Nicolai, Wolfgang, Dr. med., Am Wald 44,
D-4019 Monheim (DE)**
Erfinder: **Posselt, Klaus, Dr., Rodderbergstrasse 62,
D-5300 Bonn 2 (DE)**

(74) Vertreter: **Kraus, Walter, Dr. et al, Patentanwält Dres.
Kraus & Weisert Irmgardstrasse 15, D-8000
München 71 (DE)**

(54) Arzneimittelkombination zur Behandlung von Durchblutungsstörungen und Verfahren zu ihrer Herstellung.

(57) Die Erfindung betrifft eine Arzneimittelkombination, welche Cinnarizin bzw. ein pharmazeutisch verträgliches Salz davon und Dihydroergotamin bzw. ein pharmazeutisch verträgliches Salz davon, gegebenenfalls zusammen mit üblichen Hilfs- und Trägerstoffen, enthält. Sie kann in vorteilhafter Weise zur Behandlung von Durchblutungsstörungen verwendet werden.

**EP 0 006 435 A1**

0006435

2174 WK/li

DOLORGIET ARZNEIMITTELFABRIK PETER DOLL KG,
Bonn 2 - Bad Godesberg

Arzneimittel

Die Erfindung betrifft ein Arzneimittel zur Behandlung
von Durchblutungsstörungen.

Aus der DE-PS 1 086 235 ist 1-(Diphenylmethyl)-4-(3-phenyl-2-propenyl)-piperazin bekannt. Diese Substanz, deren
Freiname Cinnarizin ist, besitzt antihistaminische und
periphervasodilatatorische Eigenschaften.

Beispielsweise aus "The Merck Index" 1976, 3151 ist Dihydroergotamin, abgekürzt DHE, bekannt, das durch Hydrieren des natürlichen Mutterkornalkaloids Ergotamin gewonnen wird. Diese Substanz besitzt überwiegend $\alpha$-sympatho-

lytische Eigenschaften. In niedrigen Dosen wirkt sie auch kontrahierend auf die Kapazitätsgefäße.

Es wurde nun gefunden, daß Gemische aus Cinnarizin und DHE überraschende pharmakologische Eigenschaften besitzen. Besonders zur Steigerung des Blutdurchflusses zeigt die Kombination beider Verbindungen bereits in niedrigeren Dosen als die Einzelstoffe gute Wirkungen.

Eine solche Kombination aus Cinnarizin und Dihydroergotamin eignet sich daher zur Behandlung von Durchblutungsstörungen aller Art, insbesondere von peripheren und cerebralen Durchblutungsstörungen.

Gegenstand der Erfindung ist daher ein Arzneimittel, das dadurch gekennzeichnet ist, daß es Cinnarizin bzw. ein pharmazeutisch verträgliches Salz davon und Dihydroergotamin bzw. ein pharmazeutisch verträgliches Salz davon, gegebenenfalls zusammen mit üblichen Hilfs- und Trägerstoffen, enthält.

Ein weiterer Gegenstand der Erfindung ist ein Verfahren zur Herstellung eines solchen Arzneimittels, das dadurch gekennzeichnet ist, daß man die oben genannten Wirkstoffe mit üblichen pharmazeutischen Hilfs- und Trägerstoffen zu pharmazeutischen Zubereitungen verarbeitet.

Schließlich ist ein weiterer Gegenstand der Erfindung die Verwendung eines Gemisches aus Cinnarizin bzw. einem pharmazeutisch verträglichen Salz davon und Dihydroergotamin bzw. einem pharmazeutisch verträglichen Salz davon zur Behandlung von Durchblutungsstörungen.

Der Einfluß von Cinnarizin und DHE auf die periphere arterielle Durchblutung und den systolischen arteriellen Blutdruck wurde nach der Methode von H.H.Wagener und I.S. Doppelfeld, Herz-Kreislauf, 7 (6), 301-304 (1975) am mit Chloralose/Urethan narkotisierten Hund geprüft. Die Ver-

0006435

abreichung erfolgte intravenös. Der Blutfluß wurde in der rechten A. femoralis mit elektromagnetischen Strömungsaufnehmern über einen Helige-Blutströmungsmesser 236 046 01, der Blutdruck in der linken A. femoralis über ein Statham-Element P 23 Db gemessen. Beide Parameter wurden auf einem Direktschreiber, Hellige-Meßschrank AB 2002 0007, registriert (Mittelwerte aus 5 bzw. 10 Meßwerten).

| Substanz | Dosis mg/kg | Änderg.d. Durchblutung | Änderg.d. Blutdrucks | Dauer der Wirkungen min. |
|---|---|---|---|---|
| Cinnarizin | 0,316 | + 3,8 | + 3,1 | 2 |
| Cinnarzin | 1,0 | + 10,8 | − 2,3 | 6 |
| Cinnarizin | 3,16 | − 17,7 | + 10,7 | 11 |
| DHE | 0,1 | − 3,6 | + 1,2 | 3 |
| DHE | 0,316 | + 6,1 | − 3,8 | 6 |
| DHE | 1,0 | + 19,7 | − 14,2 | 12 |
| Cinnarizin + DHE | 1,0 +0,1 | + 8,4 | − 3,3 | 3 |
| Cinnarizin + DHE | 3,16 +0,316 | + 16,9 | − 29,5 | 19 |
| Cinnarizin + DHE | 5,62 +0,562 | + 23,2 | − 40,3 | 19 |

Die obige Tabelle zeigt, daß ein Gemisch aus Cinnarizin und DHE bereits in Dosen, in denen die Substanzen allein keine oder nur eine schwache Wirkung haben, eine gute Durchflußsteigerung zeigt. Es ist daher berechtigt, von einer synergistischen Wirkung der beiden Substanzen zu sprechen. Dazu kommt noch, daß die Wirkung länger anhält als im Fall der Verabreichung von Einzelsubstanzen.

Die erfindungsgemäß in Betracht gezogene Substanzkombination ist zur Herstellung pharmazeutischer Zusammensetzungen und Zubereitungen geeignet. Die pharmazeutischen Zusammensetzungen bzw. Arzneimittel enthalten als Wirkstoffe Cinnarizin bzw. ein pharmazeutisch unbedenkliches Salz davon und Dihydroergotamin bzw. ein pharmazeutisch unbedenkliches Salz davon, gegebenenfalls im Gemisch mit üblichen Hilfs- und Trägerstoffen. Als Beispiele für pharmazeutisch unbedenkliche Salze können die entsprechenden Salze mit Methansulfonsäure, Chlorwasserstoffsäure, Bromwasserstoffsäure, Schwefelsäure, Essigsäure, Weinsäure, Fumarsäure, Maleinsäure, Phosphorsäure und Ascorbinsäure genannt werden. Die Methansulfonate werden bevorzugt.

Die erfindungsgemäßen Arzneimittel können parenteral oder oral verabreicht werden, wobei die orale Applikation in Form von Tabletten, Kapseln, Dragées, Lösungen, Emulsionen und flüssigen Suspensionen bevorzugt wird.

Die Herstellung der Arzneimittel erfolgt unter Verwendung der bekannten, üblichen pharmazeutischen Hilfsstoffe sowie sonstiger üblicher Träger-, Verdünnungs-, Gleit- und Sprengmittel. Gegebenenfalls können Geschmacks- und Farbstoffe zugesetzt werden.

Als derartige Träger- und Hilfsstoffe kommen z.B. solche Stoffe in Frage, die in folgenden Literaturstellen als Hilfsstoffe für Pharmazie, Kosmetik und angrenzende Gebiete empfohlen bzw. angegeben sind: Ullmanns Encyclopädie der technischen Chemie, Band 4 (1953), Seiten 1 bis 39; Journal of Pharmaceutical Sciences, Band 52 (1963), Seite 918 ff; H. v. Czetsch-Lindenwald, Hilfsstoffe für Pharmazie und angrenzende Gebiete; Pharm. Index, Heft 2, 1961, Seite 72 ff; Dr. H. P. Fiedler, Lexikon der Hilfsstoffe für Pharmazie, Kosmetik und angrenzende Gebiete, Cantor KG, Aulendorf i. Württ., 1971.

Beispiele hierfür sind Gelatine, Rohrzucker, Pektin, Stärke, Tylose, Talkum, Lycopodium, Kieselsäure, Milchzucker, Cellulosederivate, Stearate, Emulgatoren, pflanzliche Öle, Wasser, pharmazeutisch verträgliche ein- oder mehrwertige Alkohole und Polyglykole, wie Polyäthylenglykole, sowie Derivate hiervon, Dimethylsulfoxid, Ester von aliphatischen gesättigten oder ungesättigten Fettsäuren mit ein- oder mehrwertigen Alkoholen, wie Glykolen, Glycerin, Diäthylenglykol, Pentaerythrit, Sorbit, Mannit usw., die gegebenenfalls auch veräthert sein können, Benzylbenzoat, Dioxolane, Glyzerinformale, Glycolfurole,Dimethylacetamid, Lactamide, Lactate, Äthylcarbonate usw.

Zur Herstellung von Lösungen kommen beispielsweise Wasser oder physiologisch verträgliche organische Lösungsmittel in Frage, wie z.B. Äthanol, 1,2-Propylenglykol, Polyglykole und deren Derivate, Dimethylsulfoxid, Fettalkohole, Triglyceride, Partialester des Glycerins, Paraffine und ähnliche, oder deren Mischungen.

Bei der Herstellung der Zubereitungen können bekannte und übliche Lösungsvermittler verwendet werden. Als Lösungsvermittler kommen beispielsweise in Frage: Polyoxyäthylierte Fette, polyoxyäthylierte Oleotriglyceride, linolisierte Oleotriglyceride.

Darüber hinaus ist der Zusatz von Konservierungsmitteln, Stabilisatoren, Puffersubstanzen, Geschmackskorrigentien, Antioxidantien und Komplexbildnern (z.B. Äthylendiaminotetraessigsäure) und dergl. möglich. Gegebenenfalls ist zur Stabilisierung des Wirkstoffmoleküls mit physiologisch verträglichen Säuren oder Puffern auf einen pH-Bereich von ca. 3 bis 7 einzustellen. Im allgemeinen wird ein möglichst neutraler bis schwach saurer (bis pH 5) pH-Wert bevorzugt.

Als Antioxidantien kommen beispielsweise Natriummetabisulfit, Ascorbinsäure, Gallussäure, Gallussäurealkylester, Butylhydroxyanisol, Nordihydroguajaretsäure,Tocopherole sowie Tocopherole + Synergisten (Stoffe, die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.

Als Konservierungsmittel kommen beispielsweise Sorbinsäure, p-Hydroxybenzoesäureester (z.B. Niederalkylester),Ben-

0006435

zoesäure, Natriumbenzoat, Trichlorisobutylalkohol, Phenol, Cresol, Benzethoniumchlorid und Formalinderivate in Betracht.

Die pharmakologische und galenische Handhabung der erfindungsgemäßen Kombination erfolgt nach den üblichen Standardmethoden.

Das Gewichtsverhältnis von Cinnarizin und Dihydroergotamin in der erfindungsgemäßen Kombination beträgt 10:1 bis 200:1, vorzugsweise 50:1 bis 100:1.

Bei der pharmazeutischen Anwendung liegen die Dosen für Cinnarizin zwischen 25 und 150 mg und für Dihydrogergotaminmethansulfonat zwischen 0,5 und 2,5 mg. Bevorzugt wird eine Mischung von 50 bis 100 mg Cinnarizin und 0,5 bis 1 mg Dihydroergotaminmethansulfonat, die 2- bis 3-mal täglich verabreicht werden soll.

Beispiel 1

Herstellung von Tabletten

100 g Cinnarizin und 0,5 g Dihydroergotaminmethansulfonat werden mit 35 g Alginsäure und 53 g Milchzucker gut gemischt. Anschließend wird die Pulvermischung mit einer Lösung von 10 g eines Vinylpyrrolidon-Vinylacetat-Copolymerisats (60:40) (Luviskol VA 64) in 45 ml Äthanol (96%ig) versetzt und durch ein 2 mm Sieb feucht granuliert. Das Granulat wird bei 35 bis 40°C getrocknet und anschließend durch ein 1 mm Sieb passiert. Zu dem getrockneten Granulat werden noch 40 g mikrokristalline Cellulose, 2 g Magnesiumstearat und 9,5 g Talkum gemischt. Die nun komplette Mischung wird anschließend in bekannter Weise zu Tabletten mit einem Gewicht von 250 mg und einem Durchmesser von 9 mm gepreßt. Es werden Werkzeuge mit Bruchkerbe verwendet.

0006435

1 Tablette von 250 mg Gewicht enthält:

100 mg Cinnarizin

0,5 mg Dihydroergotaminmethansulfonat

Beispiel 2

Überzogene Tabletten bzw. Dragées

Tabletten oder Dragéekerne, die entsprechend Beispiel 1 hergestellt werden, lassen sich in bekannter Weise mit einem Magen- oder Dünndarm-löslichen Filmüberzug oder mit einem Dragéeüberzug aus Saccharose, Mannit, Xylit u.a.versehen. Als Filmbildner eignen sich vorzugsweise Cellulosederivate, Copolymerisate mit kationischem Charakter auf der Basis von Dimethylaminoäthylmethylacrylat und Methacrylsäureestern bzw. Copolymerisate mit anionischem Charakter auf der Basis von Methacrylsäure und Methacrylsäureestern oder andere geeignete Filmbildner. Als geeignete Zusätze werden noch Weichmacher, Farbpigmente, Magnesiumstearat, Titandioxid usw. verwendet.

Beispiel 3

Herstellung von Gelatine-Steckkapseln

50 g Cinnarizin, 0,5 g Dihydroergotaminmethansulfonat werden mit 180 g Milchzucker (Tablettose), 10 g Alginsäure, 3,5 g Magnesiumstearat und 6 g Talkum gut gemischt. Die Mischung wird anschließend in Einzelmengen von 250 mg in Gelatine-Steckkapseln der Größe 3 abgefüllt.

1 Kapsel enthält:

50   mg Cinnarizin

0,5 mg Dihydroergotaminmethansulfonat

Beispiel 4

Herstellung einer tixotropen Suspension

1,5 g Cinnarizin und 0,01 g Dihydroergotaminmethansulfonat werden in 50 ml destilliertem Wasser gelöst bzw.suspendiert. Zu der Lösung werden anschließend 0,9 g Hydroxyäthyl-Cellulose und 2,5 mikrokristalline Cellulose gegeben und mit einem Hochleistungsrührer (Ultraturrax) 5 min. kräftig gemischt. Nach dieser Prozedur werden unter leichtem Rühren noch 52 g 70%ige Sorbitlösung, Farbstoff, Essenz und Konservierungsstoff hinzugefügt und auf ein Endvolumen von 100 ml aufgefüllt.

5 ml Suspension enthalten:
75   mg Cinnarizin
0,5 mg Dihydroergotaminmethansulfonat

P a t e n t a n s p r ü c h e

1. Arzneimittel, dadurch gekennzeichnet, daß es Cinnarizin bzw. ein pharmazeutisch verträgliches Salz davon und Dihydroergotamin bzw. ein pharmazeutisch verträgliches Salz davon, gegebenenfalls zusammen mit üblichen Hilfs- und Trägerstoffen, enthält.

2. Arzneimittel nach Anspruch 1, dadurch gekennzeichnet, daß es Cinnarizin bzw. ein pharmazeutisch verträgliches Salz davon und Dihydroergotamin bzw. ein pharmazeutisch verträgliches Salz davon im Gewichtsverhältnis von 10:1 bis 200:1, vorzugsweise 50:1 bis 100:1, enthält.

3. Arzneimittel nach Anspruch 1 oder 2, dadurch gekennzeichnet, daß es 25 bis 150 mg Cinnarizin bzw. eines pharmazeutisch verträglichen Salzes davon und 0,5 bis 2,5 mg Dihydroergotamin bzw. eines pharmazeutisch verträglichen Salzes davon, vorzugsweise 50 bis 100 mg Cinnarizin bzw. eines pharmazeutisch verträglichen Salzes davon, und 0,5 bis 1 mg Dihydroergotamin bzw. eines pharmazeutisch verträglichen Salzes davon enthält.

4. Verfahren zur Herstellung eines Arzneimittels nach den Ansprüchen 1 bis 3, dadurch gekennzeichnet, daß man die Wirkstoffe gemäß Anspruch 1 mit üblichen pharmazeutischen Hilfs- und Trägerstoffen zu pharmazeutischen Zubereitungen verarbeitet.

5. Verwendung eines Gemisches aus Cinnarizin bzw. eines pharmazeutisch verträglichen Salzes davon und Dihydroergotamin bzw. eines pharmazeutisch verträglichen Salzes davon zur Behandlung von Durchblutungsstörungen.

Europäisches
Patentamt

EUROPÄISCHER RECHERCHENBERICHT

0006435

Nummer der Anmeldung

EP 79 10 1431

| | EINSCHLÄGIGE DOKUMENTE | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | A 61 K 31/495// (A 61 K 31/495-, 31/48) |
| A | ARZNEIMITTEL-FORSCHUNG, Vol. 27(II) Nr. 8, 1977, Ed. Cantor Aulendorf, D. COSNIER: "Influence of hyper-capnia on the cerebrovascular ac- tivities of some drugs used in the treatment of cerebral ischemia", Seiten 1566-1569 <br><br> * Seite 1566, Zusammenfassung; Seite 1567, Zeilen 5,6 * <br><br> ---- | 1 | |

RECHERCHIERTE
SACHGEBIETE (Int. Cl.²)

A 61 K 31/495

KATEGORIE DER
GENANNTEN DOKUMENTE

X: von besonderer Bedeutung

A: technologischer Hintergrund

O: nichtschriftliche Offenbarung

P: Zwischenliteratur

T: der Erfindung zugrunde
liegende Theorien oder
Grundsatze

E: kollidierende Anmeldung

D: in der Anmeldung angefuhrtes
Dokument

L: aus andern Gründen
angeführtes Dokument

&: Mitglied der gleichen Patentfamilie, übereinstimmendes
Dokument

Der vorliegende Recherchenbericht wurde für alle Patentansprüche erstellt.

| Recherchenort | Abschlußdatum der Recherche | Prüfer |
|---|---|---|
| Den Haag | 02-08-1979 | BRINKMANN |

EPA form 1503.1   06.78